# EUROPÄISCHE PATENTANMELDUNG

(11) **EP 0 727 480 A2**
(43) Veröffentlichungstag der Anmeldung: **21.08.1996**
(21) Anmeldenummer: 96101940.3
(22) Anmeldetag: 10.02.1996
(51) Int. Cl.: C12M 1/40, C12M 3/00, C12M 3/06, C12M 1/04, C12Q 1/02

(54) **Verfahren zur Bestimmung von Pharmakokinetik bzw. Toxikokinetik von Testsubstanzen mit Hilfe von in-vitro-Zellkultursystemen und dafür geeignete Vorrichtungen**

(30) Priorität: 16.02.1995 DE 19505110; 05.10.1995 DE 19537033
(71) Anmelder: FORSCHUNGSZENTRUM JÜLICH GMBH, 52425 Jülich (DE)
(72) Erfinder: Papaspyrou, Manfred, Dr., D-53881 Euskirchen (DE); Biselli, Manfred, Dr., D-52428 Jülich (DE); Born, Christoph, D-52072 Aachen (DE); Schröder, Bernd, D-52428 Jülich (DE)

(57) **Zusammenfassung**

Die Erfindung betrifft ein Verfahren zur Bestimmung der Pharmakokinetik bzw. Toxikokinetik von Testsubstanzen ex vivo. Dabei wird erfindungsgemäß für die Tests eine in-vitro-Zellkultur im blasenfrei begasten Wirbelschichtreaktor verwendet. Zudem sind an Trägerkörpern Zellen angelagert, deren Anteil bezogen auf das gesamte Reaktionssystem über 1 % liegt. Des weiteren betrifft die Erfindung eine Vorrichtung zur Kultivierung menschlicher oder tierischer Organfunktionszellen, insbesondere zur Durchführung von Pharmakokinetik- bzw. Toxikokinetiktests in-vitro. Erfindungsgemäß handelt es sich bei dieser Vorrichtung um einen Wirbelschichtreaktor mit integrierter Membranbegasung, mit porösen Trägerkörpern von ≦ 1000 µm Durchmesser, Porengrößen ≦ 100 µm und einer Porösität um 50 % zur Immobilisierung der Zellen. Des weiteren beträgt das Arbeitsvolumen einschließlich Rezyklierungskreis ≦ 100 ml bei einem Schüttvolumenanteil der Trägerkörper, bezogen auf das Arbeitsvolumen, von 0,2 bis 0,6.

## Beschreibung

Die Erfindung bezieht sich auf ein Verfahren zur Bestimmung von Pharmakokinetik bzw. Toxikokinetik von Testsubstanzen ex vivo mit Hilfe von in vitro Zellkultursystemen, und sie umfaßt eine dafür geeignete Anordnung.

Jedes Jahr müssen Tausende von neuen Industriechemikalien und pharmazeutischen Wirkstoffen rigorosen Toxizitätsprüfungen unterzogen werden. Entsprechend den Richtlinien des Gesetzgebers (Chemikalien- bzw. Arzneimittelgesetz) dürfen keine Wirkstoffe ungeprüft angewendet werden. Getestet werden diese Substanzen meistens an Tieren (Mäuse, Ratten, Kaninchen, Hunde, Primaten etc.), um einen möglichen Schaden auf den menschlichen Organismus festzustellen. Die bis in die siebziger Jahre übliche Testpraxis bedeutete für die Versuchtiere z.T. erhebliche Qualen. Darüber hinaus sind Tierexperimente langwierig und extrem kostspielig. So erfolgt die Beurteilung der subakuten, subchronischen und chronischen Toxizität sowie einer möglicherweise krebserzeugenden (kanzerogene) Wirkung von Testsubstanzen derzeit, gemäß den gültigen Prüfvorschriften, mit Hilfe von Tierexperimenten. Kanzerogenitätstests gehören zu den teuersten und zeitaufwendigsten Toxizitätsprüfungen. Ihre Dauer beträgt in der Regel 24 Monate. Tests an Hunden oder Primaten können sogar 7 bis 10 Jahren dauern. Teratogene (fruchtschädigende) Wirkungen können nur im Tierexperiment untersucht werden. Aus wirtschaftlichen Überlegungen heraus und um Tierversuche auf ein Minimum zu reduzieren, wird seit Anfang der achtziger Jahre verstärkt an der Entwicklung von Ersatzmethoden gearbeitet. Mittlerweile stehen eine ganze Reihe von unterschiedlichen Systemen zur Verfügung, die in verschiedenen Teilbereichen des toxikologischen Prüfverfahrens eingesetzt werden.

Die akute Toxizität einer Prüfsubsanz kann mit Hilfe von Säugerzellkulturen durch Messung der Wachstumsverzögerung bzw. des Zellverlusts im Vergleich zu Kontrollkulturen gemessen werden (T. Lindl und J. Bauer, in Zell- und Gewebekultur, Stuttgart; New York (Fischer), 144-146, 1987). Ein weiterer in-vitro-Test funktioniert mit Hilfe eines Farbstoffes (Neutralrot), den nur lebende Zellen aufnehmen und speichern (Spektrum der Wissenschaft, 12, (1989), 94-100). Die Intensität der Neutralrotfärbung ist damit ein Maß für die Zytotoxizität der zu prüfenden Chemikalie. Substanzen bekannter Toxizität erlauben die Standardisierung dieser Testverfahren.

Zur Erfassung von mutagenen Wirkungen wird der Ames-Test (Mutation Research, 31, (1975) 347-364) als bakterielles Testverfahren routinemäßig eingesetzt. Für Gentoxizitätstests an Säugerzellkulturen haben sich zwei Tests etabliert (Ballantyne, B., et al. (Hrsg.) General and Applied Toxicology, London (Macmillan) 1993). Die Fähigkeit von Zellen, chemisch induzierte DNA-Schäden zu erkennen und zu reparieren wird im ersten Fall zur Bestimmung der Mutagenität einer Testsubstanz benutzt. Der zweite Ansatz erfaßt chromosomale Veränderungen wie Schwesterchromatidaustausche.

Eine wichtige Einflußgröße zur Entwicklung neuer Pharmaka ist insbesondere die Verteilung einer Testsubstanz im menschlichen Körper. Pharmakokinetik und Toxikokinetik beschreiben die Veränderungen von Fremdstoffkonzentrationen im Organismus. Ziel ist die quantitative Vorhersage der Stoffkonzentrationen in einzelnen Organen zu einem bestimmten Zeitpunkt. Eine pharmakokinetische Untersuchung von Testsubstanzen in vitro setzt eine, über längere Zeit unveranderte, ex vivo Kultivierung von Zellmaterialien mit organ-ähnlichem Verhalten und zwar in ausreichender Menge voraus, daß die Konzentrationsänderung im Wachstumsmedium durch Aufnahme und Verstoffwechselung der Testsubstanz durch die Zellen meßbar wird.

Die bislang bekannten Untersuchungen etwa in T-Flaschen oder in Spinnerflaschen werden beiden Forderungen kaum gerecht, d.h. weder der über längere Zeit unveränderten Aufrechterhaltung von organ-ähnlichen Mischpopulationen in vitro noch der ausreichend hohen Zellanteile pro Wachstumsmedium des Testsystems.

Ziel der Erfindung ist daher eine Biotechnik, die diesen Forderungen gerecht werden kann.

Das zu diesem Zweck entwickelte erfindungsgemäße Verfahren ist im wesentlichen dadurch gekennzeichnet, daß man für die Tests eine in vitro Zellkultur im blasenfrei begasten Wirbelschichtreaktor verwendet, mit an Trägerkörpern angelagerten Zellen, deren Anteil bezogen auf das gesamte Reaktionssystem über 1 % liegt.

Weitere Besonderheiten ergeben sich aus den Patentansprüchen und der nachfolgenden Beschreibung.

Gemäß der Erfindung wird ein in vitro System vorgesehen, mit dem die Wirkung bzw. Toxizität von Substanzen auch in zeitlichem Verlauf bzw. in Abhängigkeit von der zeitlichen Veränderung der Wirkstoffkonzentration ausreichend empfindlich bestimmt werden kann.

Fließbettreaktoren (auch als Wirbelschicht-Bioreaktoren bezeichnet) werden bereits seit einiger Zeit in der Zellkulturtechnik für die Produktion von monoklonalen Antikörpern und anderen biologisch wertvollen Stoffen verwendet. Die Nutzbarmachung dieser prinzipiell bekannten Technik für pharmakologische Untersuchungen unter Definition der notwendigen Testbedingungen hat jedoch bislang nicht stattgefunden.

Insbesondere wird erfindungsgemäß in einem Reaktor mit einem Gesamtvolumen von etwa 50 ml bei 20 bis 25 ml Träger (Schüttvolumen) gearbeitet, wobei etwa 1 bis 5 g Zellen immobilisiert werden können. Auf diese Weise können alle tierischen oder menschlichen Gewebesysteme einschließlich Tumore simuliert werden. Die Kultivierungsbedingungen lassen sich an jedes Zellmaterial (etablierte Zellinien oder Primärmaterial) optimal anpassen und manuell oder auch automatisch regulieren. Die Reaktionen des gesamten Systems können anhand einer Vielzahl von Stoffwechselparametern kontinuierlich oder chargenweise (durch Probenentnahme) gemessen und konstant gehalten oder entsprechend den experimentellen Anforderungen manipuliert werden. Aufgrund der hohen Zelldichte - pro ml Trägerschüttvolumen werden Zelldichten zwischen 5 x 10⁷ und 2 x 10⁸ Zellen erreicht; 1 cm³ tierisches Gewebe enthält je nach Ursprung zwischen 2 x 10⁸ bis 1 x 10⁹ Zellen - werden Versuchsbedingungen erreicht, die denen in einem in-vivo-System wesentlich näher kommen, als das bei allen herkömmlichen Kulturtechniken möglich ist. Dabei werden Systeme mit etwa 1 bis 10 % immobilisierten Zellen kontinuierlich erreicht, während in herkömml. Säugerzellkultursystemen wie z.B. T-Flaschen mit 80 cm² Kulturoberfläche nur 10 bis 50 mg Zellen mit 10 bis 15 ml Medium diskontinuierlich kultiviert werden können. Dies entspricht einem Zellanteil von etwa 0,1 bis 0,5 %.

Eine Bestimmung der Lebensfähigkeit der im Reaktor immobilisierten Zellen erfolgt durch die Messung ihrer Stoffwechselaktivität. Dabei wird der Verbrauch von Glukose aus dem zirkulierenden Medium sowie die Ausscheidung von Stoffwechselprodukten wie z.B. Laktat ins Medium quantitativ erfaßt. Eine toxische Wirkung einer ins zirkulierend Medium dosierten Prüfsubstanz beeinflußt die o.g. Stoffwechselparameter. Darüber hinaus verlieren adhärente Säugerzellen unter dem Einfluß einer toxischen Substanz sehr schnell die Fähigkeit, sich auf ihren Trägern festzuhalten. Wenn im um- bzw. ablaufenden Medium frei zirkulierende Zellen in großer Anzahl auftauchen, so ist dies alleine schon ein Maß für die akute Toxizität der Testsubstanz. Da freischwimmende Zellen im Abfallgefäß aufgefangen werden, kann die Zellzahl mit Hilfe eines Zellzählgerätes (Hämozytometer oder Coulter-Counter) leicht bestimmt werden. Das Ausmaß der Schädigung der Zelle kann mit Hilfe von etablierten Färbetests genauer bestimmt werden. Farbstoffe wie Trypan-Blau oder Erythrosin-B können die Membran von lebenden Zellen nicht durchdringen (Farbstoffausschlußtests). Tote Zellen haben meistens perforierte Membranen, sodaß diese Reagenzien in die Zellen eindringen können und zu einer intensiven Blau- (Trypan-Blau) bzw. Rotfärbung (Erythrosin-B) führen. Neutralrot wird hingegen nur von lebenden Zellen aufgenommen, sodaß tote Zellen farblos erscheinen.

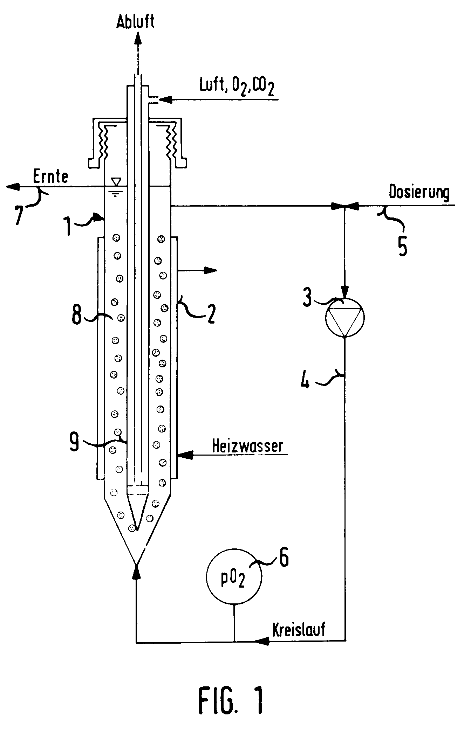

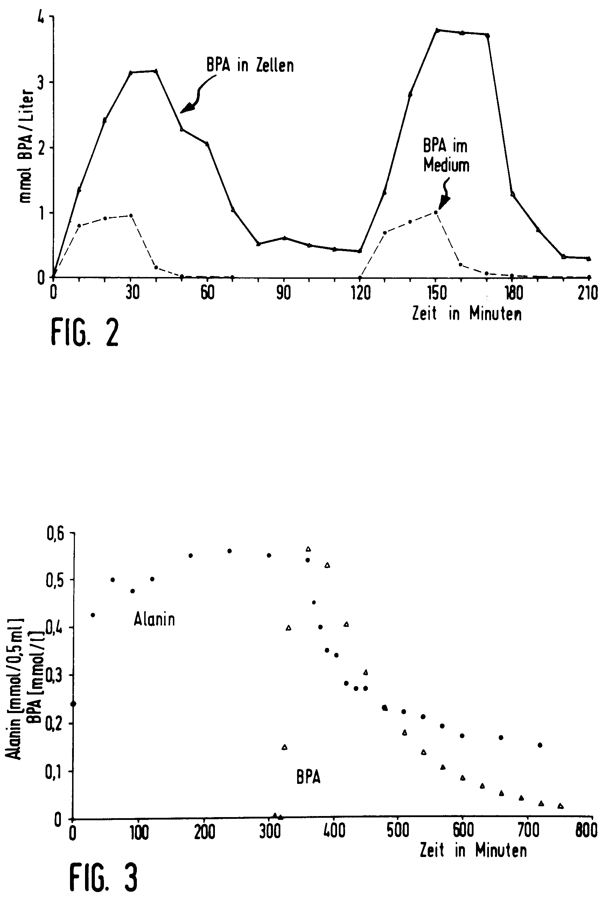

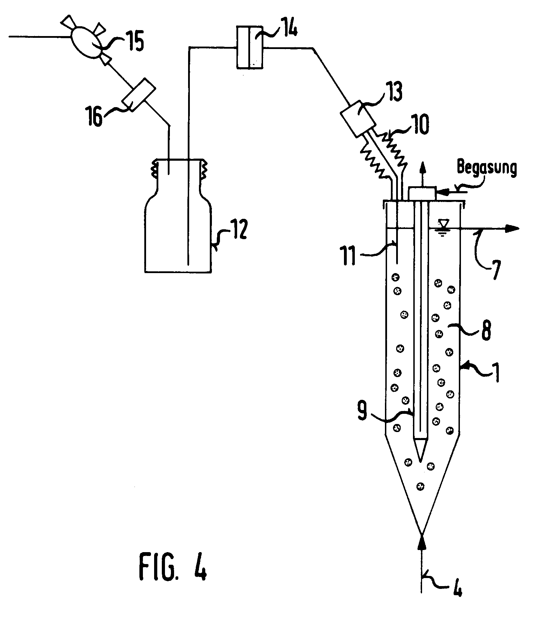

Eine Vorrichtung zur Durchführung des Verfahrens wird schematisch in den beigefügten Zeichnungen wiedergegeben; es zeigen Fig. 1 eine Anlage im Schema; Fig. 4 eine Vorrichtung zur Entnahme von Trägern aus dem laufenden Systeme; Fig.2 u.3 Kurven für die Pharmakokinetik von BPA in einem Fließbettreaktor.

Fig. 1 zeigt den Reaktor 1 mit Heizmantel 2 und mit einem von einer peristaltischen Umlaufpumpe 3 angetriebenen Rezyklierungskreis 4 für das flüssige Medium, das (schematisch mit 5 angedeutet) durch entsprechende Dosierstrategie auf optimalen Wachstums- und Erhaltungsbedingungen gehalten wird. Durch 6 wird die Überwachung des Mediums bezüglich unterschiedlicher Parameter, insbesondere mit einer Sauerstoffsonde, angedeutet. Bei 7 wird dem Reaktor Medium (z.B. durch einen Überlaufanschluß) entnommen. Zudosierung und Entnahme können auch im Kreislauf oder im Reaktor integriert sein.

Die Wirbelschicht 8 mit zellbewachsenen Trägern wird durch die unten in den Reaktor 1 einlaufende Rezyklierungsströmung im Fließzustand gehalten. Die Wirbelkörper werden durch ihr höheres, spezifisches Gewicht innerhalb der Reaktorröhre festgehalten und somit am Austrag am oberen Reaktorende in den Kreislauf gehindert. Innerhalb der zylindrischen Röhre, die das Wirbelbett enthält, ist koaxial eine Silikonmembran 9 angebracht, über die der Sauerstoff für die Zellen eingetragen wird und die im wesentlichen, wie in dem DE-GM 9413576 beschrieben, ausgebildet ist. Die Silikonmembran kann aber auch in den Rezyklierungskreislauf integriert sein.

Der Reaktor ist am unteren Ende konisch verjüngt und dem Durchmesser der Umlaufschläuche (≦ 7,5 mm) angepaßt. Das Reaktorrohr hat selbst einen Durchmesser von ca. 10-20 mm bei einem Öffnungswinkel von ca. 25°. Das Arbeitsvolumen incl. Kreislslauf betrug ∼50 ml.

Die Zellen sind vollständig oder mehrheitlich an die Oberflächen und in den Poren der porösen Wirbelkörper (0,4-0,7 mm Durchmesser; 1,1-1,3 kg/l Dichte→Poren mit Wasser gefüllt) angeheftet. Als besonders zweckmäßig haben sich Wirbelkörper aus Siran® (Firma Schott, Mainz) herausgestellt. Diese Wirbelkörper haben eine Dichte von 1,6 kg/l, der Hohlraumanteil beträgt ca. 50%. Aber alle Wirbelkörper, die das Anlagern und das Wachstum der Zellen zulassen sind innerhalb von 0,2-1,5 mm Durchmesser prinzipiell geeignet. Die Zelldichten, die insgesamt erreicht werden, liegen je nach Zelltyp zwischen 1,5 x 10⁷ und 2 x 10⁸ Zellen pro ml Wirbelkörperschüttung.

Die Entnahme von Wirbelkörperproben aus dem Reaktor ist steril (mit einer Sterilbank) über den Deckel möglich. Vorzugsweise wird ein Trägerprobenahmesystem in den Deckel integriert, wie es in Fig. 4 skizziert ist: Das Trägerprobeentnahmesystem umfaßt einen Faltenbalg 10 von variabler Länge von z.B. 12-23cm, in dem sich ein Teflonschlauch 11 befindet. Im ausgefahrenen Zustand des Faltenbalges 10 befindet sich der Teflonschlauch oberhalb des Reaktormediums im Kopf der Reaktorröhre. Somit behindert der Schlauch nicht die Strömungseigenschaften des Reaktors. Im eingefahrenen Zustand befindet sich der Schlauch im Wirbelbett, wie angezeigt. Die Trägerentnahme erfolgt durch einen Unterdruck am Schlauchende. Die Träger werden in eine Flasche 12 am Ende des Schlauches gesogen, die dann steril gewechselt wird. In der Verbindung vom Faltenbalg 10 aus flexiblem Edelstahl zur Flasche 12 befindet sich eine Reduzier- und Klemmverschraubung 13 und eine Sterilkupplung 14. Mit der Flasche 12 kann ein Peleusball 15 über ein Sterilfilter 16 verbunden sein, um auf einfache, manuelle Weise Medium und Trägerreste im Schlauch durch Überdruck zurück in den Reaktor zu befördern. Somit kann eine Trägerprobe steril aus dem Reaktor entnommen werden. Andere Entnahmesysteme sind natürl. einsetzbar.

Die Trägerprobenahmeeinrichtung wird sinnvollerweise mit dem Reaktor mit integrierter Begasungsstange kombiniert, da der Reaktordeckel hierbei während des Betriebs nicht geöffnet werden kann.

Durch die in den Reaktor integrierte Begasungsmembran ergeben sich folgende Vorteile: Gegenüber einem Reaktor mit externem Begasungsgefäß reduziert sich das Verhältnis des umlaufenden Mediums zum Schüttvolumen der Träger. Außerdem erhöht sich die Betriebssicherheit des Reaktors für den Fall, daß versehentlich Luftblasen in das laufende Rezylklierungssystem geraten. Auch kleine Luftblasen können sich am unteren Ende des Reaktorrohres zu einer einzigen Luftblase vereinigen. Wenn deren Durchmesser etwa dem des Reaktorrohres entspricht, so kann dadurch das gesamte Wirbelbett soweit angehoben werden, daß Wirbelkörper in den Rezyklierungskreislauf geraten und von der Peristaltikpumpe zerstört werden. Große Luftblasen werden an der Anstömspitze derart geteilt, sodaß die entstehenden kleinen Blasen nicht mehr in der Lage sind, daß Wirbelbett anzuheben. Die spezifische Membranfläche beträgt ca. 1 - 4 cm²/ml bei Membrandicken von 0,3 - 1 insb. 0,5 mm für die interne Begasung; bei externer Begasung im Kreilauf werden Membranflächen von etwa 10 - 20 cm²/ml Trägerschüttvolumen vorgesehen.

In der Praxis stellt sich eine Prüfung einer beliebigen Chemikalie auf akute Toxizität mit Hilfe des hier beschriebenen Systems sehr einfach dar. Ausgangssituation ist ein Reaktor, der mit vollständig besiedelten Trägern gefüllt ist und sich im Zustand eines physiologischen Fließgleichgewichts befindet. Aus einem Vorratsgefäß wird mittels einer Schlauchpumpe Frischmedium in den Reaktor gepumpt und durch die Umlaufpumpe umgewälzt. Das überschüssige (verbrauchte) Zellkulturmedium gelangt durch den Überlauf in ein Abfallgefäß. An Stelle des Abfallgefäßes kann ein Proben- bzw. Analysengefäß angeschlossen werden.

Über einen geeigneten Anschluß kann aus einer zusätzlichen Flasche über den Medienzulauf die Testsubstanz mit konstanter Konzentration und variablerer Pumpgeschwindigkeit oder bei konstanter Dosierleistung mit variabler Konzentration in den Reaktor gepumpt werden. Wenn der Testlösung ein ungiftiger Indikator (z.B. ein Lebensmittelfarbstoff oder Phenolrot) beigemischt wird, kann durch mit einem Photometer mit Durchflußküvette am Reaktorzu- bzw. Ablauf die Verweilzeit der Substanz kontinuierlich verfolgt werden. Nach beliebiger Dosierdauer kann auf Normalmedium umgeschaltet werden und durch Variation der Dosierleistung der Frischmedienpumpe eine Eliminationskinetik der Testsubstanz eingestellt werden.

Mit Hilfe der o.g. Methoden kann die Lebensfähigkeit der Zellen überwacht werden. Die Nachbeobachtung kann über mehrere Wochen ausgedehnt werden. Bei der Untersuchung der akuten Toxizität im Tierversuch erstreckt sich der Beobachtungszeitraum in der Regel nur über 2 Wochen.

Eine weitere Anwendungsmöglichkeit des hier beschriebenen Systems liegt in der Entwicklung von Chemotherapeutika gegen Tumoren. Dabei werden die auf den Trägern immobilisierten Tumorzellen als Tumorgewebe und das zirkulierende Medium als Blutkreislaufsystem betrachtet. Als Beispiel wurde die Kinetik der in vitro-Aufnahme einer borierten Aminosäure (BPA), die zur Bor-Neutroneneinfangtherapie von malignen Melanomen und Gliomen in klinischen Studien am Menschen verwendet wird, untersucht.

Für das hier beschriebene Beispiel wurde ein Reaktorsystem mit 100 ml Gesamtvolumen und 30 ml Trägern (Schüttvolumen) verwendet. Auf den Trägern wurden B16F1-Mäusemelanomzellen 2 Wochen vor Versuchsbeginn immobilisiert. Das BPA wurde als 1 millimolare Lösung mit einer Geschwindigkeit von 1 l pro Stunde für 30 Minuten in den Reaktor gepumpt. Danach wurde das BPA durch normales Zellkulturmedium ohne BPA ersetzt, bei gleichbleibender Pumpgeschwindigkeit. 120 Minuten nach Versuchsbeginn wurde wiederum für 30 Minuten BPA (1mmol/l) in den Reaktor infundiert. Anschließend wurde auch hier für 1 Stunde Normalmedium hineingepumpt. Von Beginn an wurden alle 10 Minuten Proben sowohl aus dem rezierkulierenden Medium als auch Trägerproben direkt aus der Wirbelschicht entnommen. Da jedes BPA-Molekül ein Boratom trägt, wurde die BPA-Konzentration in den Zellen und im Medium wurde mittels Elektrothermischer Atomabsorptionsspektrometrie (M.Papaspyrou et al. J. Anal.Atom.Spectr. 9 (1994) 791-795) bestimmt.

In Fig. 2 sind die Ergebnisse dieses Versuchs dargestellt. Es zeigte sich, daß die maximale BPA-Konzentration im zirkulierenden Kulturmedium (●) nach einer halben Stunde nach Versuchsbeginn bzw. nach Infusionsbeginn erreicht wurde. In den Tumorzellen (B16F1-Mäusemelanom) wurde das Maximum nach etwa einer halben bis dreiviertel Stunde erreicht. Die BPA-Konzentration in den Tumorzellen (▲) war mehr als dreimal so hoch wie die BPA-Konzentration im rezirkulierenden Medium. Nach dem Umschalten auf Normalmedium nahm die BPA-Konzentration im Umlauf viel schneller ab als in den Zellen. Nach dem erneuten Umschalten auf BPA-Medium (120 Minuten nach Versuchsbeginn) wurde ein erneuter Anstieg der BPA-Konzentration im Medium und in den Zellen beobachten. Die Konzentration in den Zellen war aber etwas höher. Da zwischen den beiden Maxima in Fig. 2 keine vollständige Elimination (Clearence) von BPA aus den Zellen stattgefunden hatte, addieren sich hier die BPA-Konzentrationen.

Somit kann einerseits die Wirksamkeit hinsichtlich der Vernichtung der Tumorzellen mit Hilfe von Dosis-Effekt-Kurven untersucht werden. Andererseits kann man mit Reaktoren, die anstelle der Tumorzellen beispielsweise Bindegewebszellen oder Epithelzellen aus verschiedenen Organen (z.B. Hepatozyten aus tierischer oder menschlicher Leber, Knochenmarkzellen, Zellen der Haut etc.) enthalten, auch die Wirkung der untersuchten Substanzen auf das Normalgewebe bestimmen. Diese Daten könnten dann eine Grundlage für tierexperimentelle Untersuchungen und darüber hinaus auch eine Basis für klinische Studien an Tumorpatienten darstellen.

Die Arbeitsbedingungen im Reaktor können so gewählt werden, daß sie mit den Bedingungen in einem in-vivo-Experiment übereinstimmen. In einem vorangegangenen Versuch (Fig.3) wurde deshalb in einen Reaktor mit 150 ml Gesamtvolumen bei 30 ml Trägervolumen 2 Wochen nach Inokulieren der Melanomzellen BPA-Medium hineingdosiert. Zum Vergleich ist die Pharmakokinetik einer Aminosäureinfusionslösung (Alanin) dargestellt, die an gesunden Probanden gemessen wurde (Ferber, H.P., und Förster, H., in Aminosäuren-Transferlösungen: Workshop d. Arbeitsgemeinschaft für künstliche Ernährung (AKE) d. Österr. Ges. für Ernährungsforschung (ÖGE) in Baden bei Wien, 8.-10. Dez. 1983/ Bd.-Hrsg.: G. Kleinberger und U. Bürger.- München, Bern, Wien: Zuckscherdt, (1985), 75-87). Das BPA-Medium wurde mit einer um das 10-fache erhöhten Dosierrate in den Reaktor gepumpt als das nachfolgende Normalmedium. Dadurch hat die BPA-Kinetik die Form einer Bolusinjektion. Bei gleicher Dosiergeschwindigkeit würde sich die Form der Kurven entsprechen.

Die Reaktionen des biologischen Materials auf eine beliebige, zu untersuchende Testsubstanz können durch Simulation der Anflutungskinetik hinsichtlich ihrer Toxizität (Toxikokinetik) und pharmakologischen Wirksamkeit (Pharmakokinetik) unter realistischen Bedingungen untersucht werden, da man das Fließbett durch die hohen Zelldichten als Gewebe betrachten und das zirkulierend Medium als Blutplasma betrachten kann.

Da eine Entnahme von kleinen Mengen Träger mit den darauf immobilisierten Zellen während der Messung möglich ist, können auch Untersuchungen zur Toxikodymamik und Pharmakodynamik an diesem System durchgeführt werden - toxiko- und pharmakodynamische Eigenschaften einer Testsubstanz bestimmen das Zielorgan und die biochemische Wirkung-.

Darüber hinaus kann eine Untersuchung von pharmakologischer Wirksamkeit eines Chemotherapeutikums und toxikologischer Nebenwirkung mit Hilfe des hier beschriebenen Ansatzes simultan an einundemselben in vitro System durchgeführt werden.

In Fig. 2 ist die Kinetik der Aufnahme von BPA in B16F1-Mäusemelanomzellen unter kontinuierlichen Bedingungen im Reaktor dargestellt. Die Entnahme von Trägern mit immobilisierten Zellen und von Proben aus dem rezirkulierenden Medium erfolgte alle 10 Minuten: (●) Zeit-Konzentrations-Profil der Zugabe von BPA im zirkulierenden Medium, (▲) Zeitliche Veränderung der Anreicherung von BPA in den Melanomzellen.

Fig. 3 zeigt Ergebnisse des Pharmakokinetiktests von BPA (▲) in einem Fließbettreaktor. Auf den Trägern waren B16F1-Mäusemelanomzellen immobilisiert. Zum Vergleich ist die Kinetik einer Aminosäureninfusion (Alanin •) bei gesunden Probanden dargestellt. 10%ige Lösung mit 160 ml/h; Infusion 6 h; Nachbeobachtung 5 h (Nach Ferber, H.P., und Förster, H., mit freundlicher Erlaubnis).

## Patentansprüche

1. Verfahren zur Bestimmung der Pharmakokinetik bzw. Toxikokinetik von Testsubstanzen ex vivo,
**dadurch gekennzeichnet,**
daß man für die Tests eine in-vitro-Zellkultur im blasenfrei begasten Wirbelschichtreaktor verwendet, mit an Trägerkörpern angelagerten Zellen, deren Anteil bezogen auf das gesamte Reaktionssystem über 1 % liegt.

2. Verfahren nach Anspruch 1,
**dadurch gekennzeichnet,**
daß mit einer in Umlauf befindlichen Flüssigkeitsmenge von ≦ 100 ml und einem Trägerkörperschüttvolumen von 20 - 60 ml von makroporösen Glasträgern mit ≦ 700 µm Durchmesser und um 50 % Porosität bei Porengrößen ≦ 100 µm gearbeitet wird.

3. Verfahren nach Anspruch 1 oder 2,
**dadurch gekennzeichnet,**
daß die Konzentration der Testsubstanz und/oder von Abbauprodukten derselben im Kulturmedium innerhalb des Reaktors und/oder im Rezyklierungskreis und/oder im Reaktorablauf überwacht wird.

4. Verfahren nach einem der vorangehenden Ansprüche,
**dadurch gekennzeichnet,**
daß die Lebensfähigkeit der Zellen über ihre Stoffwechselaktivität überwacht wird.

5. Verfahren nach einem der vorangehenden Ansprüche,
**dadurch gekennzeichnet,**
daß der Glucoseverbrauch und/oder die Lactatausscheidung in das Kulturmedium überwacht werden.

6. Verfahren nach einem der vorangehenden Ansprüche,
**dadurch gekennzeichnet,**
daß die Ausschüttung von Zellen in das Kulturmedium überwacht wird.

7. Verfahren nach einem der vorangehenden Ansprüche,
**dadurch gekennzeichnet,**
daß die Testsubstanz für eine Meßreihe durch einmalige Injektion in das Kulturmedium zugegeben wird.

8. Verfahren nach einem der vorangehenden Ansprüche,
**dadurch gekennzeichnet,**
daß die Testsubstanz kontinuierlich oder nach Programm in das Kulturmedium eingeführt wird.

9. Verfahren nach einem der vorangehenden Ansprüche,
**dadurch gekennzeichnet,**
daß die Konzentration der Testsubstanz bzw. deren Stoffwechselprodukte in den Zellen durch Entnahme von Trägerproben bestimmt wird.

10. Vorrichtung zur Kultivierung menschlicher oder tierischer Organfunktionszellen, insbesondere zur Durchführung von Pharmakokinetik- bzw. Toxikokinetik-Tests in vitro,
**gekennzeichnet durch**
einen Wirbelschichtreaktor mit integrierter Membranbegasung, mit porösen Trägerkörpern von ≦ 1000 µm Durchmesser, Porengrößen ≦ 100 µm und einer Porosität um 50 % zur Immobilisierung der Zellen und mit einem Arbeitsvolumen einschließlich Rezyklierungskreis von ≦ 100 ml bei einem Schüttvolumenanteil der Trägerkörper, bezogen auf das Arbeitsvolumen, von 0,2 bis 0,6.

11. Vorrichtung nach Anspruch 10
**gekennzeichnet durch**
eine im Reaktor koaxial angeordnete rohrförmige Begasungsmembran.

12. Vorrichtung nach Anspruch 10 oder 11,
**gekennzeichnet durch**
offenporige Glasträger als Trägerkörper.

13. Vorrichtung nach einem der Ansprüche 10 bis 12,
**gekennzeichnet durch**
Trägerkörper mit 200 bis 700 µm Durchmesser.

14. Vorrichtung nach einem der Ansprüche 10 bis 13,
**gekennzeichnet durch**
Trägerkörper mit 20 - 50 µm Porengröße.

15. Vorrichtung nach einem der Ansprüche 10 bis 14,
**gekennzeichnet durch**
einen Trägeranteil im Reaktor um 40 %, gerechnet als Schüttvolumen pro Arbeitsvolumen des Reaktors inclusive Rezyklierungskreis.
